# EUROPEAN PATENT APPLICATION

(11) **EP 4 230 217 A1**
(43) Date of publication of application: **23.08.2023**
(21) Application number: 21880631.3
(22) Date of filing: 18.10.2021
(51) Int. Cl.: A61K 38/16, A61K 47/68, A61P 37/00, A61P 29/00, A61K 38/22

(54) **PHARMACEUTICAL COMPOSITION FOR PREVENTING OR TREATING LUPUS-RELATED DISEASES COMPRISING GIP DERIVATIVE OR LONG-ACTING CONJUGATE THEREOF**

(30) Priority: 16.10.2020 KR 20200134480
(71) Applicant: Hanmi Pharm. Co., Ltd., Hwaseong-si, Gyeonggi-do 18536 (KR)
(72) Inventor: KIM, Eun Jung, Hwaseong-si, Gyeonggi-do 18469 (KR); CHOI, Jae Hyuk, Hwaseong-si, Gyeonggi-do 18469 (KR); KIM, Jeong A, Hwaseong-si, Gyeonggi-do 18469 (KR); YOO, Nyeong Sang, Hwaseong-si, Gyeonggi-do 18469 (KR); IM, Hyeon Joo, Hwaseong-si, Gyeonggi-do 18469 (KR)
(74) Representative: Brevalex
(86) International application number: PCT/KR2021/014458
(87) International publication number: WO 2022/080985

(57) **Abstract**

Provided is a pharmaceutical composition for preventing or treating lupus-associated disease, including a GIP derivative, a pharmaceutically acceptable salt or solvate thereof, or a long-acting conjugate thereof.

## Description

### Technical Field

The present disclosure relates to a pharmaceutical composition for preventing or treating lupus-associated diseases, comprising a GIP derivative or a long-acting conjugate thereof.

### Background Art

Systemic lupus erythematosus, also referred to as systemic lupus erythematodes, lupus, or S.L.E, is a chronic inflammatory autoimmune disease in which autoantibodies against specific proteins in the cell nucleus are produced and cells or tissues of oneself are destroyed by abnormalities in the immune system. In most cases, lupus has mild symptoms such as skin rashes, but in some patients, lupus can cause severe immune abnormalities in the main organs of the body such as the brain, lungs, and kidneys.

The exact cause of lupus is not known. It is only assumed that lupus is caused by a combination of genetic, hormonal, and environmental factors. In addition, ultraviolet ray exposure, silicon dioxide dust, smoking, and drugs have been reported to increase the occurrence of lupus.

The number of lupus patients is increasing every year. According to the statistics from the Health Insurance Review & Assessment Service (HIRA), 25,757 people were treated for lupus in 2017, and the number of treated people increased by about 3,000 people (13.5 %) in three years between 2015 and 2017. Regarding the number of treated people by age, the majority of patients are in their 40s (26.6 %), followed by those in their 30s (22.2 %) and those in their 50s (21.9 %). By gender, the number of female people (86.3%) was about 6.3 times higher than that of male people (13.7%). Therefore, special care seems to be required for women in their 30s to 50s.

Lupus is also classified as small vessel vasculitis. However, lupus is considered different from other vasculitis in that lupus is not only affected by a specific area, such as the blood vessel, but also targets all tissues of the body, as well as joints and muscles, such as the skin, nerve tissues, lungs, kidneys, heart, and hematopoietic organs. In the same context, in the case of lupus, since the progression of the disease is difficult to expect and various symptoms appear, lupus is difficult to diagnose and treat clinically.

A glucose-dependent insulinotropic polypeptide (GIP) is a representative hormone (incretin hormone) among those secreted from the gastrointestinal tract, and is also a neurohormone secreted in response to food intake. The GIP is a hormone consisting of 42 amino acids secreted from K cells in the small intestine, and is well known to help maintain homeostasis of blood glucose by promoting secretion of insulin or glucagon in the pancreas in a blood glucose concentration-dependent manner. Recent studies have reported diet suppression and anti-inflammatory effects of the GIP.

Meanwhile, in the case of a native GIP, the activity thereof is lost when N-terminal is cleaved by an enzyme, dipeptidyl peptidase-4 (DPP-4), and this reaction occurs at a very high speed in the body. Thus, the half-life of the GIP in the human body is known to be very short, only about 7 minutes (J Clin Endocrinol Metab. 2000 Oct;85(10):3575-81). Therefore, when utilizing efficacy of the GIP for the development of a therapeutic agent, it is required to develop a derivative having increased persistence in the body.

Accordingly, the inventors of the present disclosure developed a long-acting GIP derivative conjugate showing high activity in a human GIP receptor and having improved duration in the body, and by confirming the potential of the long-acting GIP derivative conjugate as a therapeutic agent for lupus-associated diseases, completed the present disclosure.

### Disclosure

### Technical Problem

Provided is a pharmaceutical composition for preventing or treating lupus-associated disease, comprising a GIP derivative, a pharmaceutically acceptable salt thereof, a solvate thereof, or a conjugate thereof.

Provided is a method of preventing or treating lupus-associated disease, the method comprising administering the GIP derivative, the pharmaceutically acceptable salt thereof, the solvate thereof, the conjugate thereof, or the pharmaceutical composition, in an effective amount to a subject in need thereof.

Provided is use of the GIP derivative, the pharmaceutically acceptable salt thereof, the solvate, or the conjugate for use in the preparation of a drug for preventing or treating the lupus-associated disease.

### Technical Solution

Throughout the present specification, not only the conventional 1-letter codes and 3-letter codes for amino acids present in nature, but also the 3-letter codes, such as α-aminoisobutyric acid (Aib) and the like, generally used for other amino acids, are used. In addition, the amino acids mentioned in abbreviation in the present specification are described according to the IUPAC-IUB nomenclature.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Provided is a pharmaceutical composition for preventing or treating lupus-associated disease, comprising a GIP derivative, a pharmaceutically acceptable salt thereof, or a solvate thereof.

A "glucose-dependent insulinotropic polypeptide or a gastric inhibitory polypeptide (GIP)" is a hormone secreted from K cells in the small intestine when stimulated by food intake, and was first reported as a substance involved in regulating blood sugar concentration.

The "GIP derivative" may be a native GIP in which at least one amino acid in the native GIP sequence undergoes modification. The modification may be selected from the group consisting of substitution, addition, deletion, and modification, or a combination of two or more thereof. The amino acid sequence to be added may be derived from the native GIP amino acid sequence, but is not limited thereto.

The GIP derivative may be a peptide having activity on a GIP receptor. The "peptide having activity on the GIP receptor" refers to a peptide having a significant level of activity on the GIP receptor, and specifically, having *in vitro* activity on the GIP receptor of about 0.1 % or more, about 1 % or more, about 2 % or more, about 3 % or more, about 4 % or more, about 5 % or more, about 6 % or more, about 7 % or more, about 8 % or more, about 9 % or more, about 10 % or more, about 20 % or more, about 30 % or more, about 40 % or more, about 50 % or more, about 60 % or more, about 70 % or more, about 80 % or more, about 90 % or more, about 100 % or more, or about 100 to about 500 %, or about 100 % to about 200 % compared to a native ligand (native GIP). A method of measuring the *in vitro* activity of the peptide having activity on the GIP receptor can be referred to Example 2 of the present specification, but is not particularly limited thereto. Any method known in the art may be appropriately used to measure the *in vitro* activity.

The term "about" refers to a range including all of ±0.5, ±0.4, ±0.3, ±0.2, ±0.1, etc., and includes all ranges equal to or similar to a numerical value following the term "about", but is not limited thereto.

In an embodiment, the GIP derivative may be one in which conservative substitution have occurred in 1, 2, 3, 4, 5, 6, 7, 8, 9, 10, or more amino acids in the native or unmutated GIP protein, but is not limited thereto.

The term "conservative substitution" refers to substitution of one amino acid with another amino acid having similar structural and/or chemical properties. The GIP derivative may have, for example, one or more conservative substitutions while still remaining the biological activity of the native or unmutated GIP protein. Such amino acid substitution may generally occur based on similarities in polarity, charge, solubility, hydrophobicity, hydrophilicity, and/or amphipathic nature. For example, a positively charged (basic) amino acid may include arginine, lysine, and histidine; a negatively charged (acidic) amino acid may include glutamic acid and aspartic acid; an aromatic amino acid may include phenylalanine, tryptophan, and tyrosine; and a hydrophobic amino acid may include alanine, valine, isoleucine, leucine, methionine, phenylalanine, tyrosine, and tryptophan. In addition, the amino acids may be classified into amino acids with electrically charged side chains and amino acids with uncharged side chains. Amino acids with electrically charged side chains may include aspartic acid, glutamic acid, lysine, arginine, and histidine, and amino acids with uncharged side chains may be further classified into nonpolar amino acids and polar amino acids. The nonpolar amino acid may include glycine, alanine, valine, leucine, isoleucine. methionine, proline, etc.; and the polar amino acid may include serine, threonine, cysteine, asparagine, glutamine, etc. The conservative substitution with amino acids having similar properties described above is expected to exhibit the same or similar activity of the amino acids.

The GIP derivative may be non-naturally occurring produced.

The GIP derivative may be an isolated peptide.

In an embodiment, the GIP derivative may be a peptide including the amino acid sequence represented by General Formula 1:

Tyr-Aib(aminoisobutyric acid)-Glu-Gly-Thr-Phe-lle-Ser-Asp-Tyr-Ser-lle-Xaa13-Xaa14-Xaa15-Xaa16-Xaa17-Ala-Xaa19-Xaa20-Xaa21 -Phe-Xaa23-Xaa24-Trp-Leu-Xaa27-Xaa28-Xaa29-Xaa30-Xaa31-Xaa32-Xaa33-Xaa34-Xaa35-Xaa36-Xaa37-Xaa38-Xaa39-Xaa40-Xaa41-Xaa42-Xaa43 (General Formula 1)

wherein, in General Formula 1,
Xaa13 may be alanine (Ala, A), Aib, tyrosine (Tyr, Y), or glutamine (Gln, Q),
Xaa14 may be methionine (Met, M) or leucine (Leu, L),
Xaa15 may be aspartic acid (Asp, D) or glutamic acid (Glu, E),
Xaa16 may be alanine (Ala, A), lysine (Lys, K), or glycine (Gly, G),
Xaa17 may be isoleucine (Ile, I) or glutamine (Gln, Q),
Xaa19 may be glutamine (Gln, Q) or alanine (Ala, A),
Xaa20 may be glutamine (Gln, Q), Aib, or lysine (Lys, K),
Xaa21 may be aspartic acid (Asp, D) or glutamic acid (Glu, E),
Xaa23 may be valine (Val, V) or isoleucine (Ile, I),
Xaa24 may be asparagine (Asn, N), alanine (Ala, A), or glutamine (Gln, Q),
Xaa27 may be leucine (Leu, L) or isoleucine (Ile, I),
Xaa28 may be alanine (Ala, A) or Aib,
Xaa29 may be glutamine (Gln, Q) or glycine (Gly, G),
Xaa30 may be lysine (Lys, K), glycine (Gly, G), or histidine (His, H),
Xaa31 may be proline (Pro, P), glycine (Gly, G), or cysteine (Cys, C),
Xaa32 may be serine (Ser, S) or lysine (Lys, K), or may be absent,
Xaa33 may be serine (Ser, S) or lysine (Lys, K), or may be absent,
Xaa34 may be glycine (Gly, G) or asparagine (Asn, N), or may be absent,
Xaa35 may be alanine (Ala, A) or aspartic acid (Asp, D), or may be absent,
Xaa36 may be proline (Pro, P) or tryptophan (Trp, W), or may be absent,
Xaa37 may be proline (Pro, P) or lysine (Lys, K), or may be absent,
Xaa38 may be proline (Pro, P) or histidine (His, H), or may be absent,
Xaa39 may be serine (Ser, S), asparagine (Asn, N), or cysteine (Cys, C), or may be absent,
Xaa40 may be cysteine (Cys, C) or isoleucine (Ile, I), or may be absent,
Xaa41 may be threonine (Thr, T) or may be absent,
Xaa42 may be glutamine (Gln, Q) or may be absent, and
Xaa43 may be cysteine (Cys, C) or may be absent.

Exemplary types of such a peptide may include any one amino acid sequence selected from the group consisting of SEQ ID NOs: 1 to 26.

In one or more embodiments, the peptide may include the amino acid sequence represented by General Formula 2:

Tyr-Aib(aminoisobutyric acid)-Glu-Gly-Thr-Phe-lle-Ser-Asp-Tyr-Ser-lle-Xaa13-Xaa14-Xaa15-Xaa16-Xaa17-Ala-Xaa19-Xaa20-Xaa21-Phe-Val-Xaa24-Trp-Leu-Xaa27-Xaa28-Xaa29-Xaa30-Xaa31-Xaa32-Xaa33-Xaa34-Xaa35-Xaa36-Xaa37-Xaa38-Xaa39-Xaa40-Xaa41-Xaa42-Xaa43 (General Formula 2)

wherein, in General Formula 2,
Xaa13 may be alanine (Ala, A), Aib, or tyrosine (Tyr, Y),
Xaa14 may be methionine (Met, M) or leucine (Leu, L),
Xaa15 may be aspartic acid (Asp, D) or glutamic acid (Glu, E),
Xaa16 may be alanine (Ala, A) or lysine (Lys, K),
Xaa17 may be isoleucine (Ile, I) or glutamine (Gln, Q),
Xaa19 may be glutamine (Gln, Q) or alanine (Ala, A),
Xaa20 may be glutamine (Gln, Q), Aib, or lysine (Lys, K),
Xaa21 may be aspartic acid (Asp, D) or glutamic acid (Glu, E),
Xaa24 may be asparagine (Asn, N) or glutamine (Gln, Q),
Xaa27 may be leucine (Leu, L) or isoleucine (Ile, I),
Xaa28 may be alanine (Ala, A) or Aib,
Xaa29 may be glutamine (Gln, Q) or glycine (Gly, G),
Xaa30 may be lysine (Lys, K), glycine (Gly, G), or histidine (His, H),
Xaa31 may be proline (Pro, P) or glycine (Gly, G),
Xaa32 may be serine (Ser, S) or lysine (Lys, K),
Xaa33 may be serine (Ser, S) or lysine (Lys, K),
Xaa34 may be glycine (Gly, G) or asparagine (Asn, N),
Xaa35 is alanine (Ala, A) or aspartic acid (Asp, D),
Xaa36 may be proline (Pro, P) or tryptophan (Trp, W),
Xaa37 may be proline (Pro, P) or lysine (Lys, K),
Xaa38 may be proline (Pro, P) or histidine (His, H),
Xaa39 may be serine (Ser, S), asparagine (Asn, N), or cysteine (Cys, C),
Xaa40 may be cysteine (Cys, C) or isoleucine (Ile, I), or may be absent,
Xaa41 may be threonine (Thr, T) or may be absent,
Xaa42 may be glutamine (Gln, Q) or may be absent, and
Xaa43 may be cysteine (Cys, C) or may be absent.

Exemplary types of such a peptide may include any one amino acid sequence selected from the group consisting of SEQ ID NOs: 11, 17, and 19 to 26.

In one or more embodiments, the peptide may include the amino acid sequence represented by General Formula 3:

Tyr-Aib(aminoisobutyric acid)-Glu-Gly-Thr-Phe-lle-Ser-Asp-Tyr-Ser-lle-Xaa13-Xaa14-Xaa15-Xaa16-Xaa17-Ala-Xaa19-Xaa20-Xaa21-Phe-Val-Asn-Trp-Leu-Leu-Xaa28-Xaa29-Xaa30-Xaa31-Xaa32-Xaa33-Xaa34-Xaa35-Xaa36-Xaa37-Xaa38-Xaa39-Xaa40-Xaa41-Xaa42-Xaa43 (General Formula 3)

wherein, in General Formula 3,
Xaa13 may be alanine (Ala, A) or Aib,
Xaa14 may be methionine (Met, M) or leucine (Leu, L),
Xaa15 may be aspartic acid (Asp, D) or glutamic acid (Glu, E),
Xaa16 may be alanine (Ala, A) or lysine (Lys, K),
Xaa17 may be isoleucine (Ile, I) or glutamine (Gln, Q),
Xaa19 may be glutamine (Gln, Q) or alanine (Ala, A),
Xaa20 may be glutamine (Gln, Q) or Aib,
Xaa21 may be aspartic acid (Asp, D) or glutamic acid (Glu, E),
Xaa28 may be alanine (Ala, A) or Aib,
Xaa29 may be glutamine (Gln, Q) or glycine (Gly, G),
Xaa30 may be lysine (Lys, K), glycine (Gly, G), or histidine (His, H),
Xaa31 may be proline (Pro, P) or glycine (Gly, G),
Xaa32 may be serine (Ser, S) or lysine (Lys, K),
Xaa33 may be serine (Ser, S) or lysine (Lys, K),
Xaa34 may be glycine (Gly, G) or asparagine (Asn, N),
Xaa35 is alanine (Ala, A) or aspartic acid (Asp, D),
Xaa36 may be proline (Pro, P) or tryptophan (Trp, W),
Xaa37 may be proline (Pro, P) or lysine (Lys, K),
Xaa38 may be proline (Pro, P) or histidine (His, H),
Xaa39 may be serine (Ser, S) or asparagine (Asn, N),
Xaa40 may be cysteine (Cys, C) or isoleucine (Ile, I),
Xaa41 may be threonine (Thr, T) or may be absent,
Xaa42 may be glutamine (Gln, Q) or may be absent, and
Xaa43 may be cysteine (Cys, C) or may be absent.

Exemplary types of such a peptide may include any one amino acid sequence selected from the group consisting of SEQ ID NOs: 11, 17, 21, and 24.

In one or more embodiments, in General Formula 3,
Xaa13 may be alanine (Ala, A) or Aib,
Xaa14 may be leucine (Leu, L),
Xaa15 may be aspartic acid (Asp, D) or glutamic acid (Glu, E),
Xaa16 may be lysine (Lys, K),
Xaa17 may be glutamine (Gln, Q),
Xaa19 may be glutamine (Gln, Q) or alanine (Ala, A),
Xaa20 may be glutamine (Gln, Q) or Aib,
Xaa21 may be aspartic acid (Asp, D) or glutamic acid (Glu, E),
Xaa28 may be alanine (Ala, A) or Aib,
Xaa29 may be glutamine (Gln, Q),
Xaa30 may be glycine (Gly, G) or histidine (His, H),
Xaa31 may be proline (Pro, P),
Xaa32 may be serine (Ser, S),
Xaa33 may be serine (Ser, S),
Xaa34 may be glycine (Gly, G),
Xaa35 may be alanine (Ala, A),
Xaa36 may be proline (Pro, P),
Xaa37 may be proline (Pro, P),
Xaa38 is proline (Pro, P),
Xaa39 may be serine (Ser, S),
Xaa40 may be cysteine (Cys, C), and
Xaa41 to Xaa43 may be absent.

Exemplary types of such a peptide may include any one amino acid sequence selected from the group consisting of SEQ ID NOs: 17, 21, and 24.

In one or more embodiments, in General Formula 1,
Xaa13 may be alanine (Ala, A),
Xaa14 may be methionine (Met, M),
Xaa15 may be aspartic acid (Asp, D),
Xaa16 may be alanine (Ala, A),
Xaa17 may be isoleucine (Ile, I),
Xaa19 may be glutamine (Gln, Q),
Xaa20 may be glutamine (Gln, Q),
Xaa21 may be aspartic acid (Asp, D),
Xaa23 may be valine (Val, V),
Xaa24 may be asparagine (Asn, N),
Xaa27 may be leucine (Leu, L),
Xaa28 may be alanine (Ala, A),
Xaa29 may be glutamine (Gln, Q),
Xaa30 may be lysine (Lys, K),
Xaa31 may be glycine (Gly, G),
Xaa32 may be lysine (Lys, K),
Xaa33 may be lysine (Lys, K),
Xaa34 may be asparagine (Asn, N),
Xaa35 may be aspartic acid (Asp, D),
Xaa36 may be tryptophan (Trp, W),
Xaa37 may be lysine (Lys, K),
Xaa38 may be histidine (His, H),
Xaa39 may be asparagine (Asn, N),
Xaa40 may be isoleucine (Ile, I),
Xaa41 may be threonine (Thr, T),
Xaa42 may be glutamine (Gln, Q), and
Xaa43 may be cysteine (Cys, C).

However, in General Formulae 1 to 3, when the amino acid of any one of Xaa32 to Xaa43 is absent, subsequent amino acid sequences may also be present. In an embodiment, when Xaa32 is absent, Xaa33 to Xaa43 may be absent. In one or more embodiments, when Xaa41 is absent, Xaa42 to Xaa43 may be absent.

In one or more embodiments, the peptide may include any one amino acid sequence selected from the group consisting of SEQ ID NOs: 1 to 26. In addition, the peptide may consist essentially of any one amino acid sequence selected from the group consisting of SEQ ID NOs: 1 to 26, or may consist of any one amino acid sequence selected from the group consisting of SEQ ID NOs: 1 to 26.

In one or more embodiments, the peptide may include any one amino acid sequence selected from the group consisting of SEQ ID NOs: 11, 17, and 19 to 26. In addition, the peptide may consist essentially of any one amino acid sequence selected from the group consisting of SEQ ID NOs: 11, 17, and 19 to 26, or may consist of any one amino acid sequence selected from the group consisting of SEQ ID NOs: 11, 17, and 19 to 26.

In one or more embodiments, the peptide may include any one amino acid sequence selected from the group consisting of SEQ ID NOs: 11, 17, 21, and 24. In addition, the peptide may consist essentially of any one amino acid sequence selected from the group consisting of SEQ ID NOs: 11, 17, 21, and 24, or may consist of any one amino acid sequence selected from the group consisting of SEQ ID NOs: 11, 17, 21, and 24.

In one or more embodiments, the peptide may include any one amino acid sequence selected from the group consisting of SEQ ID NOs: 17, 21, and 24. In addition, the peptide may consist essentially of any one amino acid sequence selected from the group consisting of SEQ ID NOs: 17, 21, and 24, or may consist of any one amino acid sequence selected from the group consisting of SEQ ID NOs: 17, 21, and 24.

Although described as 'a peptide consisting of a particular SEQ ID NO' herein, such expression does not exclude a mutation that can occur by a meaningless sequence addition upstream or downstream of the amino acid sequence of the corresponding SEQ ID NO, or a silent mutation therein, as long as the peptide having such mutation has activity the same as or corresponding to that of the peptide which consists of the amino acid sequence of the corresponding SEQ ID NO. Even when the sequence addition or a mutation is present, it obviously belongs to the scope of the present disclosure. That is, even there are differences in some sequences of the peptide, the peptide belongs to the scope of the present disclosure as long as the sequence identity of at least a certain level is shown and the activity on the GIP receptor is exhibited. In detail, the peptide may include the amino acid sequence having a sequence identity of 40 %, 45 %, 50 %, 55 %, 60 %, 65 %, 70 %, 75 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or 99 % or more with the amino acid sequences of SEQ ID NOs: 1 to 26, but is not limited thereto.

The term "homology" or "identity" as used herein refers to relatedness between two amino acid sequences or base sequences given, and is expressed as a percentage. Whether any two peptide sequences have homology, similarity, or identity may be determined by using known computer algorithms, such as the "FASTA" program using, for example, the default parameters as in Pearson et al. (1988)[Proc. Natl. Acad. Sci. USA 85]: 2444. Alternatively, the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (5.0.0 version. or later) may be used for the determination (other programs include the GCG program package (Devereux, J., et al, Nucleic Acids Research 12: 387 (1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the BLAST of the National Center for Biotechnology Information database or ClustalW may be used to determine homology, similarity, or identity.

The homology, similarity, or identity of peptides may be, for example, determined by comparing sequence information using a GAP computer program, such as Needleman et al.(1970), J Mol Biol.48: 443, as described in Smith and Waterman (Adv. Appl. Math (1981) 2:482). Briefly, the GAP program defines homology, similarity, or identity as the number of aligned symbols (i.e., amino acids), which are similar, divided by the total number of symbols in the shorter of the two sequences. Default parameters for the GAP program may include: (1) a unary comparison matrix (containing a value of 2 for identities and 0 for non-identities) and the weighted comparison matrix of Gribskov et al(1986) Nucl. Acids Res. 14: 6745, as described by Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4); (2) a penalty of 3.0 for each gap and an additional 0.10 penalty for each symbol in each gap (or a gap opening penalty of 10 and a gap extension penalty of 0.5); and (3) no penalty for end caps. Therefore, the term "homology" or "identity" used herein refers to relevance among sequences.

In an embodiment, the peptide including the amino acid sequence of General Formula 1 according to an aspect may be prepared by combining several methods for the preparation of various peptides.

The peptide according to an aspect may be synthesized by, depending on a length of the peptide, a method well known in the art, e.g., an automatic peptide synthesizer, and may be produced by genetic engineering technology. In detail, the peptide may be prepared by a standard synthesis method, a recombinant expression system, or any other method known in the art. Accordingly, the peptide according to an aspect may be prepared by various methods including, for example, the methods
described below, but the methods are not limited thereto:
   (a) a method of synthesizing a peptide by a solid-phase or liquid-phase method stepwise or by fragment assembly, followed by isolation and purification of the final peptide product; or
   (b) a method of expressing a nucleic acid construct encoding a peptide in a host cell and recovering the expression product from the host cell culture; or
   (c) a method of performing an *in vitro* cell-free expression of a nucleic acid construct encoding a peptide and recovering the expression product therefrom; or
a method of obtaining peptide fragments by any combination of the methods (a), (b), and (c), obtaining the peptide by linking the peptide fragments, and then recovering the peptide.

In addition, the preparation of the peptide may include all of the modifications using L-type or D-type amino acids, and/or non-native type amino acids; and/or a modification of native sequence, for example, modification of a functional group on a side chain, an intramolecular covalent bonding, e.g., a ring formation between side chains, methylation, acylation, ubiquitination, phosphorylation, aminohexanation, biotinylation, etc. In addition, the modification may include substitutions into non-native compounds.

For the amino acids to be substituted or added during the modification, not only the 20 amino acids commonly found in human proteins, but also atypical or non-naturally occurring amino acids may be used. Commercial sources of atypical amino acids may include Sigma-Aldrich, ChemPep, and Genzyme pharmaceuticals, but are not limited thereto. For example, Aib(aminoisobutyric acid) may be prepared by using Strecker amino acid synthesis starting from acetone, but the synthesis method is not limited thereto. The peptides including these atypical or non-naturally occurring amino acids and typical peptide sequences may be synthesized and purchased from commercial peptide synthesis company, e.g., American Peptide Company or Bachem in USA or Anygen in Korea, but embodiments are not limited thereto.

In addition, the peptide may have an unmodified N-terminus and/or C-terminus. However, a peptide modified in a way that the N-terminus and/or C-terminus or the like is chemically modified or protected with an organic group to protect from proteinases *in vivo* and to increase stability, or that an amino acid is added to the terminus of the peptide also belongs to the peptide according to the one aspect. When the C-terminus is not modified, the terminus of the peptide may have a free carboxyl group, but embodiments are not particularly limited thereto.

In particular, in the case of the chemically synthesized peptide, the N- and C-terminus thereof are charged, and thus the N-terminus and/or C-terminus may be modified to remove these charges. For example, the N-terminus may be acetylated and/or the C-terminus may be amidated, but embodiments are not particularly limited thereto.

In an embodiment, the C-terminus of the peptide may not be modified or may be amidated, but embodiments are not limited thereto.

In one embodiment, the peptide may include the C-terminus that is amidated.

The peptide may include all forms of the peptide itself, a salt thereof (e.g., a pharmaceutically acceptable salt of the peptide), or a solvate thereof.

Types of the salt are not particularly limited. However, a salt in a form that is safe and effective for a subject, e.g., a mammal, is preferable, embodiments are not particularly limited thereto.

In addition, the peptide may be in any form that is pharmaceutically acceptable.

The term "pharmaceutically acceptable" as used herein refers to an amount sufficient to exhibit a therapeutic effect and not causing a side effect, and such an amount may easily be determined by one of ordinary skill in the art depending on factors well known in medical fields, such as a type of disease, age, weight ,health, and gender of a patient, sensitivity of a patient to drug, a route of administration, a method of administration, the number of administration, a treatment period, a combination of drugs, or drugs used simultaneously.

In an embodiment, the peptide may be in the form of a pharmaceutically acceptable salt thereof. The salt may include conventional acid addition salts used in the pharmaceutical field, such as in the field of lupus therapeutic agents. Examples of the acid addition salt are: a salt derived from inorganic acid such as hydrochloric acid, bromic acid, sulfuric acid, sulfamic acid, phosphoric acid, or nitric acid; and a salt derived from an organic acid such as acetic acid, propionic acid, succinic acid, glycolic acid, stearic acid, citric acid, maleic acid, malonic acid, methanesulfonic acid, tartaric acid, maleic acid, phenylacetic acid, glutamic acid, benzoic acid, salicylic acid, 2-acetoxybenzoic acid, fumaric acid, toluenesulfonic acid, oxalic acid, or trifluoroacetic acid. In addition, the salt may be a base addition salt such as ammonium, dimethylamine, monomethylamine, monoethylamine, and diethylamine. In addition, the salt may include a common metal salt form, for example, a salt derived from metal such as lithium, sodium, potassium, magnesium, or calcium. The acid addition salt, the base addition salt, or the metal salt may be prepared according to a conventional method. A pharmaceutically acceptable salt and a general methodology for preparing the same are well known in the art. For example, the document [P. Stahl, et al. Handbook of Pharmaceutical Salts: Properties, Selection and Use, 2nd Revised Edition (Wiley-VCH, 2011)]; [S.M. Berge, et al., "Pharmaceutical Salts," Journal of Pharmaceutical Sciences, Vol. 66, No. 1, January 1977] may be referred.

For condensation of the protected amino acid or peptide, various activating reagents useful in peptide synthesis, particularly preferably, a trisphosphonium salt, a tetramethyluronium salt, carbodiimide, and the like may be used. Examples of the trisphosphonium salt are benzotriazole-1-yl-oxy-tris(pyrrolidino)phosphonium hexafluorophosphate(PyBOP), brom-otris(pyrrolidino)phosphonium hexafluorophosphate (PyBroP), 7-azabenzotriazole-1-yl-oxytris(pyrrolidino)phosphonium hexafluorophosphate(PyAOP), examples of the tetramethyluronium salt are 2-(1H-benzotriazol-1-yl)-1,1,3,3-tetramethyluronium hexafluorophosphate (HBTU), 2-(7-azabenzotriazol-1-yl)-1,1,3,3-tetramethyluronium hexafluorophosphate(HATU), 2-(1H-benzotriazol-1-yl)-1,1,3,3-tetramethyluronium tetrafluoroborate (TBTU), 2-(5-norborene-2,3-dicarboxyimide)-1,1,3,3-tetramethyluronium tetrafluoroborate (TNTU), and O-(N-succinimidyl)-1,1,3,3-tetramethyluronium tetrafluoroborate (TSTU), and examples of the carbodiimide are N,N'-dicyclohexylcarbodiimide (DCC), N,N'-diisopropylcarbodiimide (DIPCDI), N-ethyl-N'-(3-dimethylaminopropyl)carbodiimide hydrochloric acid (EDCl·HCl), and the like. For condensation using these salts, addition of racemizing inhibitors [e.g., N-hydroxy-5-norbornene-2,3-dicarboxylic acid imide(HONB), 1-hydroxybenzotriazole (HOBt), 1-hydroxy-7-azabenzotriazole (HOAt), 3,4-dihydro-3-hydroxy-4-oxo-1,2,3-benzotriazine (HOOBt), ethyl 2-cyano-2-(hydroxyimido)acetate (Oxyma), etc] may be preferable. A solvent used for the condensation may be appropriately selected from those known to be useful for peptide condensation reactions. For example, amide such as anhydrous or water-containing N,N-dimethyl formamide, N,N-dimethylacetamide, N-methylpyrrolidone , etc., halogenated hydrocarbon such as methylene chloride, chloroform, etc., alcohol such as trifluoroethanol, phenol, etc., sulfoxide such as dimethylsulfoxide, tertiary amine such as pyridine, etc., ether dioxane, tetrahydrofuran , etc., nitrile such as acetonitrile, propionitrile, etc., ester such as methyl acetate, ethyl acetate, etc., an appropriate mixture thereof, and the like may be used. A temperature for reactions may be appropriately selected from the range known to be available for the peptide binding reaction, and may be generally selected from the range of about -20 °C to about 90 °C. Activated amino acid derivatives may be generally used in excess of 1.5- to 6-fold. In the solid-phase synthesis, when a test using a ninhydrin reaction indicates that the condensation is insufficient, sufficient condensation can be performed by repeating the condensation reaction without removing the protecting group. When the condensation is still insufficient even after repeating the reaction, unreacted amino acid may be acetylated with an acid anhydride, acetylimidazole, etc., so that the influence on subsequent reactions can be avoided.

Examples of a protecting group for amino group of starting amino acid include benzyloxycarbonyl (Z), tert-butoxycarbonyl Boc), tert-pentyloxycarbonyl, isobonyloxycarbonyl, 4-methoxybenzyloxycarbonyl, 2-chlorobenzyloxycarbonyl(CI-Z), 2-bromobenzyloxycarbonyl(Br-Z), adamantyloxycarbonyl, trifluoroacetyl, phthaloyl, formyl, 2-nitrophenylsulphenyl, diphenylphosphinothioyl, 9-fluorenylmethyloxycarbonyl (Fmoc), trityl, and the like.

Examples of carboxyl-protecting group for starting amino acid are, in addition to the C₁₋C₆ alkyl group, the C₃₋C₁₀ cycloalkyl group, and the C₇₋C₁₄ aralkyl group described above, aryl, 2-adamantyl, 4-nitrobenzyl, 4-methoxybenzyl, 4-chlorobenzyl, fenacil, and benzyloxycarbonyl hydrazide, tert-butoxycarbonyl hydrazide, tritylhydrazide, and the like.

The hydroxyl groups of serine or threonine may be, for example, protected by esterification or etherification. Examples of groups suitable for esterification are a lower (C₂₋C₄)alkynoyl group, such as an acetyl group, an aroyl group, such as a benzoyl group, and a group derived from an organic acid, and the like. In addition, examples of groups suitable for etherification are benzyl, tetrahydropyranyl, tert-butyl (Bu^{t}), trityl (Trt), and the like.

Examples of a protecting group for a phenolic hydroxyl group of thyrosine are Bzl, 2,6-dichlorobenzyl, 2-nitrobenzyl, Br-Z, tert-butyl, and the like.

Examples of a protecting group for imidazole of histidine are toluenesulfonyl (Tos), 4-methoxy-2,3,6-trimethylbenzenesulfonyl (Mtr), dinitrophenyl (DNP), benzyloxymethyl (Bom), tert-butoxymethyl (Bum), Boc, Trt, Fmoc, and the like.

Examples of a protecting group for a guanidino group of arginine are Tos, Z, 4-methoxy-2,3,6-trimethylbenzenesulfonyl (Mtr), p-methoxybenzenesulfonyl (MBS), 2,2,5,7,8-pentamethylchroman-6-sulfonyl (Pmc), mesithylene-2-sulfonyl (Mts), 2,2,4,6,7-pentamethyldihydrobenzofuran-5-sulfonyl (Pbf), Boc, Z, NO₂, and the like.

Examples of a protecting group for an amino group in side chains of lysine Z, Cl-Z, trifluoroacetyl, Boc, Fmoc, Trt, Mtr, 4,4-dimethyl-2,6-dioxocyclohexylidenyl (Dde), and the like.

Examples of a protecting group for indolyl of tryptophan are formyl (For), Z, Boc, Mts, Mtr, and the like.

Examples of a protecting group for asparagine and glutamine are Trt, xantyl (Xan), 4,4'-dimethoxybenzhydryl (Mbh), 2,4,6-trimethoxybenzyl (Tmob), and the like.

Examples of an activated carboxyl group among the starting materials corresponding anhydride acid, an azide group, activity ester[ester with alcohol (e.g., pentachlorophenol, 2,4,5-trichlorophenol, 2,4-dinitrophenol, cyanomethylalcohol, paranitrophenol, HONB, N-hydroxysuccinimide, 1-hydroxybenzotriazole (HOBt), 1-hydroxy-7-azabenzotriazole (HOAt))], and the like. Examples of an activated amino group in the starting material are phosphorus amide and the like.

Examples of a method of removing the protecting group are: catalytic reduction in a hydrogen stream in the presence of a catalyst such as Pd-black or Pd-carbon; acid treatment with anhydrous fluorinated hydrogen, methanesulfonic acid, trifluoromethanesulfonic acid, trifluoroacetic acid (TFA), trimethylsilyl bromide (TMSBr), trimethylsilyl trifluoromethanesulfonate, tetrafluoroboronic aicd, tris(trifluoro)boronic acid, boron triboromide, or a mixture solution thereof; base treatment using diisopropylethylamine, triethylamine, piperidine, piperazine, etc.; and reduction with sodium in liquid ammonia. The aforementioned removal reaction by acid treatment may be generally performed at a temperature in a range of -20 °C to 40 °C, and the acid treatment may be efficiently performed by adding: anisole, phenol, thioanisole, metacresol, and paracresol; a cation scavenger, such as dimethylsulfide, 1,4-butanedithiol, 1,2-ethanedithiol, triisopropylsilane, etc. In addition, the 2,4-dinitrophenyl group used as the protecting group for histidine may be removed by thiophenol treatment; and the formyl group used as the protecting group for indole of tryptophan may be removed by deprotection not only by acid treatment in the presence of 1,2-ethanedithiol, 1,4-butanedithiol, and the like, but also by alkali treatment with dilute sodium hydroxide, dilute ammonia, and the like.

Protection of a functional group that should not be involved in the reaction between the starting material and the protecting group, removal of the protecting group, activation of a functional group involved in the reaction, and the like may be appropriately selected from known protecting groups and known methods.

For the peptide as used herein, the left end is the N-terminus (amino terminus) and the right end is the C-terminus (carboxyl terminus) according to conventional peptide markings. The C-terminus of the peptide may be any one of amide (-CONH₂), carboxyl group (-COOH), carboxylate (-COO-), alkylamide (-CONHR', wherein R' is alkyl), and ester (-COOR', wherein R' is alkyl or aryl).

In the method of preparing amide of the peptide, amide may be formed by solid-phase synthesis using a resin for amide synthesis, or by amidation of the α-carboxyl group of a carboxy-terminal amino acid. Then, the peptide chain is extended toward the amino group to a desired chain length, and afterwards, a peptide from which the protecting group for the N-terminal α-amino group of the peptide chain is removed and a peptide from which only the protecting group for the C-terminal carboxyl group is removed from the peptide chain are prepared. These two peptides are then condensed in the mixed solvent described above. For details of the condensation reaction, the same description above can be applied the same. After the protected peptide obtained by the condensation is purified, all protecting groups may be removed by the method described above, so as to obtain a desired peptide. By purifying the peptide using various publicly known methods of purification and the major fraction and freeze-drying, desired amide of the peptide may be prepared.

In an embodiment, the peptide may be in the form of a solvate of the peptide. The term "solvate" as used herein refers that the peptide or a salt thereof form a complex with a solvent molecule.

In an embodiment, the peptide may be in the form of a long-acting conjugate. The conjugate may exhibit activity on GIP receptors equal to or greater than that of native GIP, and at the same time, may exhibit increased efficacy of duration compared to that of a native GIP or GIP derivative to which a carrier is not linked. Therefore, the conjugate may be a long-acting conjugate. The term "long-acting conjugate" as used herein refers to a conjugate which exhibits an enhanced efficacy of duration compared to that of a native GIP or GIP derivative to which a biocompatible material is not linked. Therefore, the conjugate may be referred to as "a long-acting GIP derivative conjugate" or "a long-acting GIP derivative," or "a long-acting GIP conjugate". Such a conjugate may include not only the aforementioned forms, but also a form encapsulated in biodegradable nanoparticles.

The conjugate may be an isolated conjugate.

The conjugate may be a non-naturally occurring conjugate.

In an embodiment, the peptide may be in the form of a conjugate to which a biocompatible material that increases the half-life *in vivo* is conjugated. Therefore, the pharmaceutical composition may include a conjugate in which the GIP derivative is conjugated with a biocompatible material that increases the half-life *in vivo.* The biocompatible material may be used interchangeably with a carrier.

The biocompatible material be linked to the GIP derivative through a covalent chemical bond or a non-covalent chemical bond, and may be linked thereto via a linker (L) by the covalent chemical bond, the non-covalent chemical bond, or a combination thereof. One or more side chains of amino acids in the GIP derivative may be conjugated to the biocompatible material to increase *in vivo* solubility and/or *in vivo* half-life and/or to increase bioavailability. Such modifications may also reduce clearance of therapeutic proteins and peptides. The aforementioned biocompatible material may be water soluble (amphiphilic or hydrophilic) and/or non-toxic and/or pharmaceutically acceptable.

The biocompatible material may be selected from the group consisting of a high-molecular polymer, fatty acid, cholesterol, albumin and a fragment thereof, an albumin-binding material, a polymer of repeating units of specific amino acid sequences, an antibody, an antibody fragment, an FcRn-binding material, *in vivo* connective tissue, a nucleotide, fibronectin, transferrin, a saccharide, heparin, and elastin, but embodiments are not particularly limited thereto.

Examples of the high-molecular polymer are polyethylene glycol (PEG), polypropylene glycol, an ethylene glycol-propylene glycol copolymer, polyoxyethylated polyol, polyvinylalcohol, disaccharide, polyvinylethylether, a biodegradable polymer, a lipid polymer, chitin, hyaluronic acid, an oligonucleotide, and a combination thereof. The disaccharide may include dextran, but embodiments are not particularly limited thereto.

The PEG is a term including all types of an ethylene glycol homopolymer, a PEG copolymer, or an mPEG, but embodiments are not particularly limited thereto.

The fatty acid may have a binding affinity to albumin *in vivo,* but embodiments are not particularly limited thereto.

The biocompatible material may include poly-amino acids such as poly-lysine, poly-aspartic acid, and poly-glutamic acid, but embodiments are not limited thereto.

In the case of elastin, elastin may be human tropoelastin, which is a watersoluble precursor, and may be a polymer of some sequences or some repeating units of tropoelastin, including, for example, all types of elastin-like polypeptides, but embodiments are not particularly limited thereto.

In an embodiment, the biocompatible material may be an FcRn-binding material. In detail, the FcRn-binding material may be an immunoglobulin Fc region, and in one or more embodiments, may be an IgG Fc region, and in one or more embodiments, may be a non-glycosylated IgG4 Fc region, but embodiments are not particularly limited thereto.

The term "immunoglobulin Fc region" as used herein refers to a region including the heavy chain constant region 2 (CH2) and/or the heavy chain constant region 3 (CH3), excluding the heavy chain and light chain variable regions of an immunoglobulin. The immunoglobulin Fc region may be one constitution that establishes a moiety of the conjugate according to an aspect.

Such an immunoglobulin Fc region may include a hinge region in the heavy chain constant region, but embodiments are not limited thereto.

In an embodiment, the immunoglobulin Fc region may include a specific hinge sequence at the N-terminus.

The term "hinge sequence" as used herein refers to a site located in the heavy chain to form a dimer of immunoglobulin Fc fragments through an inter disulfide bond.

In an embodiment, the hinge sequence may be mutated to have only one cysteine residue by deletion a part of the hinge sequence having the following amino acid sequences , embodiments are not particularly limited thereto:
Glu-Ser-Lys-Tyr-Gly-Pro-Pro-Cys-Pro-Ser-Cys-Pro (SEQ ID NO: 27).

The hinge sequence may include only one cysteine residue by deletion of the 8th or 11th cysteine residue of the hinge sequence of SEQ ID NO: 27. The hinge sequence according to an embodiment may consist of 3 to 12 amino acids including only one cysteine residue, but embodiments are not particularly limited thereto. In detail, the hinge sequence according to an embodiment may have the following sequence: Glu-Ser-Lys-Tyr-Gly-Pro-Pro-Pro-Ser-Cys-Pro (SEQ ID NO: 28), Glu-Ser-Lys-Tyr-Gly-Pro-Pro-Cys-Pro-Ser-Pro (SEQ ID NO: 29), Glu-Ser-Lys-Tyr-Gly-Pro-Pro-Cys-Pro-Ser (SEQ ID NO: 30), Glu-Ser-Lys-Tyr-Gly-Pro-Pro-Cys-Pro-Pro (SEQ ID NO: 31), Lys-Tyr-Gly-Pro-Pro-Cys-Pro-Ser (SEQ ID NO: 32), Glu-Ser-Lys-Tyr-Gly-Pro-Pro-Cys (SEQ ID NO: 33), Glu-Lys-Tyr-Gly-Pro-Pro-Cys (SEQ ID NO: 34), Glu-Ser-Pro-Ser-Cys-Pro (SEQ ID NO: 35), Glu-Pro-Ser-Cys-Pro (SEQ ID NO: 36), Pro-Ser-Cys-Pro (SEQ ID NO: 37), Glu-Ser-Lys-Tyr-Gly-Pro-Pro-Ser-Cys-Pro (SEQ ID NO: 38), Lys-Tyr-Gly-Pro-Pro-Pro-Ser-Cys-Pro (SEQ ID NO: 39), Glu-Ser-Lys-Tyr-Gly-Pro-Ser-Cys-Pro (SEQ ID NO: 40), Glu-Ser-Lys-Tyr-Gly-Pro-Pro-Cys (SEQ ID NO: 41), Lys-Tyr-Gly-Pro-Pro-Cys-Pro (SEQ ID NO: 42), Glu-Ser-Lys-Pro-Ser-Cys-Pro (SEQ ID NO: 43), Glu-Ser-Pro-Ser-Cys-Pro (SEQ ID NO: 44), Glu-Pro-Ser-Cys (SEQ ID NO: 45), Ser-Cys-Pro (SEQ ID NO: 46).

In more detail, the hinge sequence may include the amino acid sequence of SEQ ID NO: 37 (Pro-Ser-Cys-Pro) or SEQ ID NO: 46 (Ser-Cys-Pro), but embodiments are not particularly limited thereto.

The immunoglobulin Fc region according to an embodiment may be in a form in which two molecules of the immunoglobulin Fc chain form a dimer due to the presence of the hinge sequence. In addition, in the conjugate of Formula 1 according to an embodiment, one end of the linker may be linked to one chain of the immunoglobulin Fc region that is in a dimeric form, but embodiments are not limited thereto.

The term "N-terminus" as used herein refers to the amino terminus of a protein or polypeptide, and may include the most end of the amino terminus or 1, 2, 3, 4, 5, 6, 7, 8, 9, or 10 or more amino acids from the most end of the amino terminus. The immunoglobulin Fc fragment of the present disclosure may include a hinge sequence at the N-terminus, but embodiments are not particularly limited thereto.

In addition, the immunoglobulin Fc region may be an extended Fc region including a part or the entirety of the heavy chain constant region 1 (CH1) and/or the light chain constant region (CL1), excluding the heavy chain and light chain variable regions of the immunoglobulin, as long as the immunoglobulin Fc region has an effect substantially the same as or improved compared to the native type. In addition, the immunoglobulin Fc region may be a region in which a fairly long part of the amino acid sequence corresponding to CH2 and/or CH3 is removed.

For example, the immunoglobulin Fc region may be selected from the group consisting of: (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one or two or more domains among a CH1 domain, a CH2 domain, a CH3 domain, and a CH4 domain, and an immunoglobulin hinge region or a part of the hinge region; and (f) a dimer between each domain of the heavy chain constant region and the light chain constant region, but embodiments are not limited thereto.

The immunoglobulin Fc region may be in a dimeric form, and one molecule of the GIP derivative may be covalently linked to a single Fc region in a dimeric form. Here, the immunoglobulin Fc and the GIP derivative may be interlinked by a non-peptide polymer. In addition, two molecules of the GIP derivative may be possibly conjugated in a symmetrical manner to a single Fc region in a dimeric form. Here, the immunoglobulin Fc and the GIP derivative may be interlinked by a non-peptide linker. However, embodiments are not limited thereto.

In addition, the immunoglobulin Fc region may include not only a native amino acid sequence, but also a sequence derivative thereof. An amino acid sequence derivative refers to an amino acid sequence having a difference in at least one amino acid residue among the native amino acid sequence due to deletion, insertion, non-conservative or conservative substitution, or a combination thereof.

For example, in the case of IgG Fc, amino acid residues at positions 214 to 238, 297 to 299, 318 to 322, or 327 to 331, which are known to be in the binding, may be used as suitable sites for modification. In addition, other various types of derivatives may be available in a that a site where a disulfide bond can be formed is deleted, some amino acid residues at the N-terminus of native Fc are deleted, or a methionine residue is added at the N-terminus of native Fc. In addition, to remove effector functions, a complement-binding site, such as a C1q-binding site, may be deleted, and an antibody dependent cell mediated cytotoxicity (ADCC) site may be deleted. Techniques of preparing such sequence derivatives of the immunoglobulin Fc region are disclosed in International Patent Publication Nos. WO 97/34631 and WO 96/32478.

Amino acid exchanges in proteins and peptides, which do not entirely alter the activity of the molecules, are known in the art (H.Neurath, R.L.Hill, The Proteins, Academic Press, New York, 1979). The most commonly occurring exchanges are exchanges between amino acid residues, such as Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, phosphorylation, sulfation, acrylation, glycosylation, methylation, farnesylation, acetylation, amidation, and the like may be used for the modification.

The aforementioned Fc derivative shows the same biological activity to that of the Fc region, and may have improved structural stability against heat, pH, etc.

In addition, the Fc region may be obtained from native forms isolated *in vivo* from humans or animals such as cows, goats, pigs, mice, rabbits, hamsters, rats, guinea pigs, etc., or may be recombinants or derivatives thereof obtained from transformed animal cells or microorganisms. Here, the obtaining of the Fc region in a native form may be a method of isolating a whole immunoglobulin from a living human or animal body and treating the isolated immunoglobulin with a protease. When treated papain, the immunoglobulin may be cleaved into Fab and Fc, or when treated with pepsin, the immunoglobulin may be cleaved into pF'c and F(ab)₂. The Fc or pF'c may be isolated by using size-exclusion chromatography, etc. In a more specific embodiment, a human-derived Fc region may be a recombinant immunoglobulin Fc region obtained from a microorganism.

In addition, the immunoglobulin Fc region may have natural glycans, increased glycans compared to the natural type, or decreased glycans compared to the natural type, or may be in a deglycosylated form. For the increase, decrease, or removal of the glycans of the immunoglobulin Fc, conventional methods such as a chemical method, an enzymatic method, and a genetic engineering method using a microorganism may be used. Here, the immunoglobulin Fc region in which the glycans are removed from Fc may have a significantly decreased binding affinity to the complement (c1q), and reduced or removed antibody-dependency cytotoxicity or complement-dependency cytotoxicity, and thus unnecessary immune responses *in vivo* are not caused. In this regard, an immunoglobulin Fc region in a deglycosylated or aglycosylated form may be more suitable to meet the original purpose as a drug carrier.

The term "deglycosylation" as used herein refers to an Fc region from which sugars are removed by enzymes, and the term "aglycosylation" as used herein refers to an unglycosylated FC region produced in prokaryotes, more specifically, E. coli.

In addition, the immunoglobulin Fc region may be derived from IgG, IgA, IgD, IgE, IgM, or a combination or hybrid thereof. In a detailed embodiment, the immunoglobulin Fc region may be derived from IgG or IgM, which are among the most abundant proteins in human blood, and in a more detailed embodiment, the immunoglobulin Fc region may be derived from IgG which is known to enhance the half-lives of ligand-binding proteins. In a more detailed embodiment, the immunoglobulin Fc region may be an IgG4 Fc region, and in a most specific embodiment, the immunoglobulin Fc region may be an aglycosylated Fc region derived from human IgG4, but embodiments are not limited thereto.

The term "combination" as used herein refers that polypeptides encoding single-chain immunoglobulin Fc regions of the same origin are linked to a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

The GIP derivative may be linked to a biocompatible material through a linker.

The linker may be a peptide linker or a non-peptide linker.

When the linker is a peptide linker, the linker may include at least one amino acid, for example, 1 amino acid to 1,000 amino acids, but embodiments are not particularly limited thereto. The peptide linker may include Gly, Asn, and Ser residues, and may also include neutral amino acids such as Thr and Ala. For the linkage between the biocompatible material and the GIP derivative, various known peptide linkers may be used. In addition, the number of copies "n" may be adjusted in consideration of linker optimization to achieve proper separation between functional moieties or to maintain essential inter-moiety interactions. Other soluble linkers are known in the art, and for example, a GS linker, in which not only a polar amino acid residue is added to improve water solubility, but also amino acid residues, such as T and A, are added to maintain flexibility may be used. Therefore, in an embodiment, the linker may be a flexible linker including G, S, and/or T residues. The linker may have a general formula selected from (GpSs)n and (SpGs)n, wherein, independently, p may be an integer of 1 to 10, s may be an integer of 0 to 10, the sum of p and S may be an integer of 20 or less, and n may be an integer of 1 to 20. In detail, the linker may have, for example, a general formula of (GGGGS)n, (SGGGG)n, (SRSSG)n, (SGSSC)n, (GKSSGSGSESKS)n, (RPPPPC)n, (SSPPPPC)n, (GSTSGSGKSSEGKG)n, (GSTSGSGKSSEGSGSTKG)n, (GSTSGSGKPGSGEGSTKG)n, or (EGKSSGSGSESKEF)n, wherein n may be an integer of 1 to 20 or an integer of 1 to 10.

The "non-peptide linker" may include a biocompatible polymer in which at least two repeating units are linked. These repeating units may be linked with each other by any covalent bond instead of a peptide linkage. The non-peptide linker may be one constitution that establishes a moiety of the conjugate.

The terms "non-peptide linker" and "non-peptide polymer" may be used interchangeably.

In an embodiment, in the conjugate, the biocompatible material and the GIP derivative may be covalently linked through a non-peptide linker which includes a reactive group that can be linked to the biocompatible material, such as the immunoglobulin Fc region, and the GIP derivative at both ends of the conjugate, respectively.

In detail, the non-peptide linker may be selected from the group consisting of a peptide, a fatty acid, a saccharide, a high-molecular polymer, a low-molecular compound, a nucleotide, and a combination thereof.

Although not particularly limited thereto, the non-peptide linker may be selected from the group consisting of PEG, polypropylene glycol, an ethylene glycol-propylene glycol copolymer, polyoxyethylated polyol, polyvinylalcohol, a polysaccharide, polyvinyl ethyl ether, a biodegradable polymer such as polylactic acid (PLA) and polylactic-glycolic acid (PLGA), a lipid polymer, chitins, hyaluronic acid, an oligonucleotide, and a combination thereof. The polysaccharide may be dextran, but embodiments are not limited thereto.

In a more specific embodiment, the non-peptide polymer may be polyethylene glycol, embodiments are not particularly limited thereto. Therefore, the linker may include an ethylene glycol repeating unit. In addition, derivatives that are already known in the art and derivatives that can be easily prepared at the level of technology in the art belong to the scope of the present disclosure.

For use as the non-peptide linker, any polymer having a resistance to *in vivo* proteases may be used without limitation. The formula weight of the non-peptide polymer may be in a range of 1 kDa to 1,000 kDa, for example, 1 kDa to 100 kDa, and for example, 1 kDa to 20 kDa, but embodiments are not particularly limited thereto. In addition, the non-peptide linker may include not only a single type of a polymer but also a combination of different types of polymers. In an embodiment, the formula weight of the ethylene glycol repeating unit may be in a range of 1 kDa to 100 kDa, for example, 1 kDa to 20 kDa.

In an embodiment, both ends of the non-peptide linker may each be linked to the biocompatible material, e.g., an amine group or a thiol group of the immunoglobulin Fc region, and an amine group or a thiol group of the GIP derivative.

In an embodiment, the non-polymer may include a reactive group at both ends thereof, respectively, which can be linked to the biocompatible material (e.g., the immunoglobulin Fc region) and the GIP derivative, specifically, an reactive group that can be linked to an amine group located at the N-terminus or lysine, or a thiol group of cysteine of the GIP derivative or the biocompatible material (e.g., the immunoglobulin Fc region) , but embodiments are not particularly limited thereto.

In addition, the reactive group of the non-peptide polymer, which can be linked to the biocompatible material, e.g., the immunoglobulin Fc region, and the GIP derivative may be selected from the group consisting of an aldehyde group, a maleimide group, and a succinimide derivative, embodiments are not particularly limited thereto. In the above, an example of the aldehyde group is a propionaldehyde group or a butylaldehyde, but embodiments are not particularly limited thereto. In the above, as the succinimide derivate, succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, or succinimidyl carbonate may be used, but embodiments are not particularly limited thereto.

In addition, the final product produced through reductive alkylation via an aldehyde bond may be more stable than that linked by an amide bond. The aldehyde reactor selectively reacts with the N-terminus at a low pH, and may form a covalent bond with a lysine residue at high pH, e.g., pH 9.0.

In addition, the reactive groups at both ends of the non-peptide linker may be identical to or different from each other. For example, a maleimide group may be provided at one end and an aldehyde group, a proprionaldehyde group, or a butyl aldehyde group may be provided at the other end. However, as long as the biocompatible material, specifically, the immunoglobulin Fc region and the GIP derivative, can be conjugated at each end of the non-peptide linker, embodiments are not particularly limited thereto. For example, the non-peptide linker may include, as the reactive group, a maleimide group as the reactive group at one end and an aldehyde group, a propionaldehyde group, a butylaldehyde group, etc. at the other end.

When PEG is used as the non-peptide polymer having a reactive hydroxy group at both ends, the hydroxy group may be activated to various reactive groups by known chemical reactions, or PEG having a commercially available modified reactive group may be used to prepare the long-acting conjugate.

In an embodiment, the non-peptide polymer may be one which can be linked to a cysteine residue of the GIP derivative, and more specifically, to a -SH group of cysteine, embodiments are not particularly limited thereto.

When maleimide-PEG-aldehyde is used, the maleimide group may be linked to the -SH group of the GIP derivative by a thioether and the aldehyde group may be linked to the biocompatible material, specifically, a -NH₂ group of the immunoglobulin Fc through reductive alkylation. However, embodiments are not limited thereto, and the above is merely an exemplary embodiment.

In addition, in the conjugate, the reactive group of the non-peptide polymer may be linked to -NH₂ located at the N-terminus of the immunoglobulin Fc region, but this embodiment is merely an exemplary embodiment.

Therefore, the conjugate according to an aspect may be represented by Formula 1:

Formula 1 X-L-F

wherein, in the formula above, X is the GIP derivative,
L is a linker,
F is a biocompatible material that increases the half-life of X *in vivo,* and
- represents a bond between X and L and a bond between L and F.

In Formula 1, the GIP derivative, the linker, and the biocompatible material are the same as described above.

In Formula 1, L may be La, wherein a is 0 or a natural number, and when a is 2 or more, each of L(s) may be independent of each other.

In detail, the linker may be PEG represented by Formula 2, but embodiments are not limited thereto: 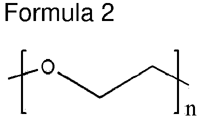 wherein n may be 10 to 2400, n may be 10 to 480, or n may be 50 to 250, but embodiments are not particularly limited thereto.

In the long-acting conjugate, a PEG moiety may include not only a -(CH₂CH₂O)n-structure but also an oxygen atom between an linking element and the-(CH₂CH₂O)n-, but embodiments are not limited thereto.

The PEG is a term including all types of an ethylene glycol homopolymer, a PEG copolymer, or an mPEG, but embodiments are not particularly limited thereto.

In an embodiment, - may represent a covalent bond linkage between X and L and a covalent bond linkage between L and F.

The GIP derivative or the conjugate thereof is confirmed to improve edema, restore skin lesions, and reduce the weight and size of the spleen that has been enlarged due to an inflammatory response, in lupus disease model mice. Therefore, the GIP derivative or the conjugate thereof may be utilized for use in the prevention or treatment of lupus-associated disease.

The term "prevention" as used herein refers to all kinds of actions associated with the suppression or delay of the occurrence of lupus-associated disease by the administration of the composition.

The term "treatment" as used herein refers to all kinds of actions associated with the improvement or advantageous changes of lupus-associated disease by the administration of the composition.

The "lupus" is autoimmune disease. Inflammation appears throughout the body when immune cells (leukocytes) of the body attack the body and cause damage on tissue. Lupus refers to a red rash, and is called systemic lupus erythematosus, or lupus for short, since this disease occurs not only on the skin but also on the whole body. 90 % of lupus patients are women, and the onset of this disease is in the childbearing age between the ages of 20 and 50. Since lupus is autoimmune disease, inflammation occurs anywhere in the body, and accordingly, symptoms appear in various ways and the symptoms change over time, and thus lupus is a disease that is not easy to diagnose.

Autoimmune responses can cause inflammation anywhere in the body, and depending on where inflammation is caused, a variety of symptoms are generated. When listed the symptoms in order of common occurrence, inflammation in the joints causes joint pain, systemic inflammation causes fever throughout the body, and dermatitis causes erythema. Proteinuria occurs due to inflammation of the kidneys, and chest pain occurs due to inflammation of the membranes surrounding the lungs and heart. In addition, photosensitive responses, hair loss, blood cell abnormalities, Raynaud's phenomenon, convulsions, mouth ulcers, and the like may occur.

The "lupus" generally refers to systemic lupus erythematosus. The term "systemic lupus erythematosus (SLE)", which is also referred to as "systemic lupus erythematosus" or "S.L.E", is a chronic inflammatory autoimmune disease, and is a systemic disease invading various organs of the body including the connective tissues, skin, joints, blood, kidneys, etc. The SLE does not necessarily show symptoms throughout the body, and there may be mild cases in which symptoms appear only in a part of the body. Lupus is also classified as a type of small vessel vasculitis. However, unlike vasculitis that only affects a specific area called blood vessel, lupus targets all tissues throughout the body. Therefore, drugs that are effective in treating vasculitis are not necessarily effective in treating lupus, and vice versa.

The term "lupus-associated diseases" includes lupus or SLE in the ordinary meaning, and is meant to include all diseases or symptoms associated with or related to lupus. The lupus-associated diseases may refer to the appearance of symptoms in any one or more organs of the lungs, heart, muscles, and joints. The lupus-associated disease may be associated with skin lesions. Thus, the lupus-associated disease can be diagnosed by skin lesion score. The pharmaceutical composition according to an embodiment may have an effect of treating lupus-associated diseases by reducing skin lesions of a patient.

In detail, the lupus-associated diseases may include SLE, discoid lupus erythematosus (DLE), drug-induced lupus, neonatal lupus, and the like.

The "discoid lupus erythematosus (DLE)" is also referred to as chronic discoid lupus erythematodes" or "cutaneous lupus erythematosus", and causes rashes on the skin of face and limbs. By lapse of time, follicular plugging, pigmentation, or the like may be accompanied.

The "drug-induced lupus" refers to lupus caused by administration of a specific drug.

The "neonatal lupus" refers to lupus that occurs in newborns born to mothers who suffered from lupus during pregnancy.

In addition, the lupus-associated diseases may include, for example, diseases associated with lupus, such as lupus nephritis, pericarditis, pleuritis, interstitial pneumonia, arthritis, and the like.

In addition, the lupus-associated diseases may include, for example, symptoms related to lupus, such as butterfly rash, pernio-like rash, Raynaud's phenomenon, oral ulcer, photosensitivity, livedo, and the like.

In an embodiment, the lupus-associated diseases may include SLE, DLE, drug-induced lupus, neonatal lupus, lupus nephritis, butterfly rash, or pernio-like rash, but embodiments are not limited thereto.

In an embodiment, the pharmaceutical composition may reduce the skin lesion. The pharmaceutical composition may reduce skin lesion scores that have been increased by lupus-associated diseases.

The pharmaceutical composition may further include a pharmaceutically acceptable carrier. The pharmaceutically acceptable carrier may include, for oral administration, a binder, a glidant, a disintegrant, an excipient, a solubilizer, a dispersant, a stabilizer, a suspending agent, a colorant, and a flavoring agent, and the like; for injections, a buffer, a preservative, an analgesic, a solubilizer, an isotonic agent, a stabilizer, and the like; and for topical administration, a base, an excipient, a lubricant, and a preservative, and the like.

In an embodiment, the pharmaceutical composition may further include a pharmaceutically acceptable excipient.

The formulation type of the pharmaceutical composition may be prepared variously by combining with the aforementioned pharmaceutically acceptable carrier. For example, for oral administration, the pharmaceutical composition may be formulated into tablets, troches, capsules, elixirs, suspensions, syrups, wafers, etc. For injections, the pharmaceutical composition may be formulated into a single dosage ampoule or a multiple dosage form. Additionally, the pharmaceutically composition may be formulated into solutions, suspensions, tablets, capsules, and sustained-release formulations.

Meanwhile, examples of suitable carriers, excipients, and diluents for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, or mineral oil. In addition, the pharmaceutical composition may further include a filler, an anticoagulant, a lubricant, a humectant, a flavoring agent, an emulsifier, a preservative, etc.

The pharmaceutical composition may further include one or more other agents for treating the lupus-associated diseases. In detail, the other agents may include an anti-inflammatory agent or an immunosuppressive agent, but embodiments are not particularly limited thereto. In more detail, the other agents may include a therapeutic agent for lupus, but embodiments are not particularly limited thereto.

The term "anti-inflammatory agent" as used herein refers to a compound for treating an inflammatory disease or a symptom related thereto. Non-limiting examples of the anti-inflammatory agents include: but not limited thereto, a non-steroidal anti-inflammatory drug (NSAID; e.g., aspirin, ibuprofnaproxen, methyl salicylate, diflunisal, indometacin, sulindac, diclofenac, ketoprofen, ketorolac, carprofen, fenoprofen, mefenamic acid, piroxicam, meloxicam, methotrexate, celecoxib, valdecoxib, parecoxib, etoricoxib, and nimesulide), corticosteroid (e.g., prednisone, betamethasone, budesonide, cortisone, dexamethasone, hydrocortisone, methyl prednisolone, prednisolone, triamcinolone, and fluticasone), rapamycin (e.g., see document [Migita et al., Clin. Exp. Immunol. (1997) 108:199-203]; [Migita et al., Clin. Exp. Immunol. (1996) 104:86-91]; [Foroncewicz et al., Transpl. Int. (2005) 18:366-368]), a compound increasing levels of high-density lipoprotein (HDL) and HDL-cholesterol (e.g., see documents [Birjmohun et al. (2007) Arterioscler. Thromb. Vasc. Biol., 27:1153-1158]; [Nieland et al. (2007) J. Lipid Res., 48:1832-1845]; [Bloedon et al. (2008) J. Lipid Res., Samaha et al. (2006) Arterioscler. Thromb. Vasc. Biol., 26:1413-1414] disclosing use of rosiglitazone as an anti-inflammatory agent; and [Duffy et al. (2005) Curr. Opin. Cardiol., 20:301-306]), a rho-kinase inhibitor (e.g., see document [Hu, E. (2006) Rec. Patents Cardiovasc. Drug Discov., 1:249-263]), an antimalarial drug (e.g., hydroxychloroquine and chloroquine), acetaminophen, glucocorticoid, steroid, beta-agonist, anticholinergic, methyl xanthine, gold injection (e.g., sodium aurothiomalate), sulfasalazine, penicillamine, antiangiogenic drug, dapsone, psoralen, antiviral drug, statin (e.g., see document [Paraskevas et al. (2007) Curr. Pharm. Des., 13:3622-36]; [Paraskevas, K.I. (2008) Clin. Rheumatol. 27:281-287]), and an antibiotic (e.g., tetracycline). In a specific embodiment, the anti-inflammatory agent may be statin or a compound increasing levels of HDL and HDL-cholesterol.

The "immunosuppressant" and "immunosuppressive agent" may include compounds or compositions that suppress the immune response or symptoms related thereto. Non-limiting examples of the immunosuppressant are a purin analogue (e.g., azathioprine), methotrexate, cyclosporine (e.g., cyclosporine A), cyclophosphamide, leflunomide, mycophenolate (mycophenolate mofetil), steroid (e.g., glucocorticoid and corticosteroid), methylprednisone, prednisone, a non-steroidal anti-inflammatory drug (NSAID), chloroquine, hydroxychloroquine, chlorambucil, a CD20 antagonist (e.g., rituximab, ocrelizumab, beltuzumab, or ofatumumab), abatacept, a TNF antagonist (e.g., infliximab, adalimumab, and etanercept), macrolide (e.g., pimecrolimus, tacrolimus (FK506), and sirolimus), dehydroepiandrosterone, lenalidomide, a CD40 antagonist (e.g., an anti-CD40L antibody), abetimus sodium, a BLys antagonist (e.g., anti-BLyS (e.g., belimumab)), dactinomycin, bucillamine, penicillamine, leflunomide, mercaptopurine, a pyrimidine analogue (e.g., cytosine arabinoside), mizoribine, an alkylating agent (e.g., nitrogen mustard, phenylalanine mustard, busulfan, and cyclophosphamide), a folic acid antagonist (e.g., aminopterin and methotrexate), an antibiotic (e.g., rapamycin, actinomycin D, mitomycin C, puramycin, and chloramphenicol), human IgG, anti-lymphocyte globulin (ALG), an antibody (e.g., anti-CD3(OKT3), anti-CD4(OKT4), anti-CD5, anti-CD7, an anti-IL-2 receptor (e.g., daclizumab and basiliximab), anti-alpha/beta TCR, anti-ICAM-1, murononab-CD3, anti-IL-12, alemtuzumab, and an antibody to an immunotoxin), 1-methyltryptophan, and a derivative and analogue thereof. In a specific embodiment, the immunosuppressant may be selected from the group consisting of methotrexate, hydroxychloroquine, a CD20 antagonist (e.g., rituximab, ocrelizumab, beltuzumab, or ofatumumab), abatacept, a TNF antagonist (e.g., infliximab, adalimumab, and etanercept), sirolimus, and a BLyS antagonist (e.g., anti-BLyS (e.g., belimumab)).

The term "therapeutic agent for lupus" as used herein may include a compound or composition that inhibits or treats symptoms associated with lupus. For the therapeutic agent for lupus, substances known in the art may be used.

The dosage and number of administration of the pharmaceutical composition may be determined according to the type of drug as an active ingredient, together with various related factors such as a disease to be treated, a route of administration, an age, a gender, and a weight of a patient, and severity of disease.

Since the pharmaceutical composition has excellent *in vivo* persistence and potency, the number and frequency of administration may be significantly reduced.

Another aspect provides a method of preventing or treating lupus-associated disease, the method comprising administering the GIP derivative, the pharmaceutically acceptable salt thereof, the solvate thereof, the conjugate thereof, or the pharmaceutical composition, in an effective amount to a subject in need thereof.

The GIP derivative, the pharmaceutically acceptable salt or solvate thereof, the conjugate, the pharmaceutical composition, and the lupus-associated disease are the same as described above.

The term "effective amount" or "pharmaceutically effective amount" as used herein refers to an amount or quantity of the GIP derivative, the pharmaceutically acceptable salt or solvate thereof, or the conjugate, which can provide a desired effect to a patient under diagnosis or treatment when administered in a single dose or multiple doses. The effective amount may be readily determined by an attending diagnostician as a person skilled in the art by using known techniques or by observing results obtained under similar circumstances. When determining the effective amount for a patient, the mammalian species; the body size, age, and general health conditions of a patient; the specific disease or disorder involved; the degree or severity of involvement of the disease or disorder; the responsiveness in individual patients; the specific compound to be administered; the administration mode; the bioavailability characteristics of an agent to be administered; the selected dosing regimen; use of concomitant medication; and other relevant circumstances may be considered, but number of factors not limited thereto are also considered by an attending diagnostician.

The term "subject" as used herein refers to a target in need of treatment for a disease, and more particularly, to a mammal including a human or a non-human primate, such as a mouse, a rat, a dog, a cat, a horse, a cow, and the like.

The term "administration" as used herein refers to introduction of a given substance to a patient by any suitable method. The route of administration may be any general route capable of reaching a target *in vivo* in a patient. The administration may be, for example, intraperitoneal administration, intravenous administration, intramuscular administration, subcutaneous administration, intradermal administration, oral administration, topical administration, intranasal administration, or intrarectal administration, but embodiments are not limited thereto.

The composition according to an embodiment may be administered, at a daily rate per subject, in a range of 0.0001 mg to 1,000 mg, for example, 0.1 mg to 1,000 mg, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, or 1 mg to 25 mg. However, the dosage may be variously prescribed depending on factors, such as a formulation method, an administration method, age, weight, gender, and medical conditions of a patient, food, administration time, an administration route, an excretion rate, and response sensitivity, and a person skilled in the art may appropriate adjust the dosage in consideration of these factors. The number of administration may be once a day or at least twice a day within the range of clinically acceptable side effects, and the administration may be performed at a single site or at least two sites, daily or every 2 days to 5 days. The total number of administration days may be 1 day to 30 days per treatment. As needed, the same treatment may be repeated after a suitable period of time. For animals other than humans, the same dosage per kg as for humans may be used, or for example, a dosage converted from the aforementioned dosage by the volume ratio (e.g., average value) of the organ (e.g., heart, etc.) between a target animal and the human may be used.

In the method, the effective amount of the GIP derivative, the pharmaceutically acceptable salt or solvate thereof, or the conjugate thereof may be administered simultaneously, separately, or sequentially with the effective amount of one or more other active ingredients. The one or more other active ingredients may include one or more other agents for treating the lupus-associated disease, but embodiments are not limited thereto.

Another aspect provides use of the GIP derivative, the pharmaceutically acceptable salt or solvate thereof, or the conjugate to be utilized in the preparation of a drug for preventing or treating lupus-associated disease.

The GIP derivative, the pharmaceutically acceptable salt or solvate thereof, the conjugate, and the lupus-associated disease are the same as described above.

Descriptions and embodiments disclosed herein may also be applied to other descriptions and embodiments, respectively. That is, all combinations of various elements disclosed herein belong to the scope of the present disclosure. In addition, the scope of the present application is not construed to be limited by the detailed description provided below.

### Advantageous Effects

A GIP derivative according to an aspect or a long-acting conjugate thereof may have effects of improving edema, restoring skin lesions, and reducing the weight and size of the spleen that has been enlarged due to an inflammatory response, in lupus disease model mice, and thus can be used for the purpose of preventing or treating lupus-associated disease.

### Description of Drawings

FIG. 1 is a diagram showing results of SDS-PAGE analysis on GIP derivative (SEQ ID NOs: 11, 17, 21, and 24)-PEG-immunoglobulin Fc region conjugates.
FIG. 2 is a graph showing the weight change rate (%) over 10 weeks in a normal mouse control group, a disease model mouse (MRL/Ipr) control group, and a group administered with a long-acting GIP conjugate.
FIG. 3 is a graph showing the skin lesion scores after 10 weeks of administration in a normal mouse control group, a disease model mouse (MRL/Ipr) control group, and a group administered with a long-acting GIP conjugate.
FIG. 4A is a graph showing the spleen weight after 10 weeks of administration in a normal mouse control group, a disease model mouse (MRL/Ipr) control group, and a group administered with a long-acting GIP conjugate.
FIG. 4B shows the spleen size after 10 weeks of administration in a normal mouse control group, a disease model mouse (MRL/Ipr) control group, and a group administered with a long-acting GIP conjugate.

### Best Mode

Hereinafter, the present disclosure will be described in detail with reference to Examples below. However, these Examples are for illustrative purposes only, and the scope of the present disclosure is not intended to be limited by these Examples.

### Example 1: Preparation of GIP derivative having activity on GIP receptor

GIP derivatives exhibiting activity on human GIP receptors were prepared, and sequences thereof are shown in Table 1.

**Table 1**

| SEQ ID NO. | Amino acid sequence |
|---|---|
| 1 | YAibEGT FISDY SIAMD AIAQQ DFVNW LLAQK PSSGA PPPSC |
| 2 | YAibEGT FISDY SIAMD AIAQQ DFVNW LLAGG PSSGA PPPSC |
| 3 | YAibEGT FISDY SIAibMD AIAQQ DFVNW LLAGG PSSGA PPPSC |
| 4 | YAibEGT FISDY SIYMD AIAQQ DFVNW LLAGG PSSGA PPPSC |
| 5 | YAibEGT FISDY SIQMD AIAQQ DFVNW LLAGG PSSGA PPPSC |
| 6 | YAibEGT FISDY SIYLD AIAQQ DFVNW LLAGG PSSGA PPPSC |
| 7 | YAibEGT FISDY SIYLD AQAQQ DFVNW LLAGG PSSGA PPPSC |
| 8 | YAibEGT FISDY SIYLD AQAAAib DFVNW LLAGG PSSGA PPPSC |
| 9 | YAibEGT FISDY SIYLD AQAAK DFVNW LLAGG PSSGA PPPSC |
| 10 | YAibEGT FISDY SIYLD AQAAK EFIAW LLAGG PSSGA PPPSC |
| 11 | YAibEGT FISDY SIAMD AIAQQ DFVNW LLAQK GKKND WKHNI |
| | TQC |
| 12 | YAibEGT FISDY SIYLD KQAAAib EFVNW LLAQK GKKND WKHNI TQC |
| 13 | YAibEGT FISDY SIYLD KQAAAib EFVNW LLAQK C |
| 14 | YAibEGT FISDY SIYLD AQAAAib EFVNW LLAQK C |
| 15 | YAibEGT FISDY SIAMD KIAQQ DFVNW LLAQK PSSGA PPPSC |
| 16 | YAibEGT FISDY SIAMD GIAQQ DFVNW LLAQK PSSGA PPPSC |
| 17 | YAibEGT FISDY SIALE KQAQQ DFVNW LLAGG PSSGA PPPSC |
| 18 | YAibEGT FISDY SIYLD KQAAQ EFVNW LLAQK PSSGA PPPSC |
| 19 | YAibEGT FISDY SIYLD KQAAAib EFVNW LLAibGH PSSGA PPPSC |
| 20 | YAibEGT FISDY SIYLD KQAAAib EFVNW LLAibGG PSSGA PPPSC |
| 21 | YAibEGT FISDY SIAibLD KQAAAib EFVNW LLAibGG PSSGA PPPSC |
| 22 | YAibEGT FISDY SIYLD KQAAAib EFVNW LLAGH PSSGA PPPSC |
| 23 | YAibEGT FISDY SIYLD KQAQK EFVNW LLAibGG PSSGA PPPSC |
| 24 | YAibEGT FISDY SIAibLD KQAAAib EFVNW LLAibGH PSSGA PPPSC |
| 25 | YAibEGT FISDY SIYLD KQAAAib EFVQW LIAibGG PSSGA PPPSC |
| 26 | YAibEGT FISDY SIYLD KQAAAib EFVQW LIAGH PSSGA PPPC |

In the amino acid sequences in Table 1, the amino acid indicated as Aib refers to aminoisobutyric acid (Aib) which is a non-natural amino acid.

The GIP derivative peptide may be used as a GIP derivative of which the C-terminus is amidated, as necessary.

### Example 2: Measurement of in vitro activity of GIP derivative

To measure the activity of the GIP derivatives prepared in Example 1, a method of measuring cell activity *in vitro* using a cell line transformed with the GIP receptor was used. The cell line was transformed to express each of human GIP receptor genes in the Chinese hamster ovary (CHO), and are suitable for the measurement of GIP activity.

To measure the activity of the GIP derivatives prepared in Example 1 in the human GIP receptors, human GIP was serially diluted from 16 nM to 0.000015 nM by 4 folds, and the GIP derivatives prepared in Example 1 were each serially diluted from 16 nM to 0.000015 nM by 4 folds. The culture medium was removed from the cultured CHO cells where the human GIP receptors were expressed, 5 µl of each of the serially diluted materials was added to the cells, and then, 5 µl of buffer containing cAMP antibody was added thereto, followed by incubation at room temperature for 15 minutes. Next, the cells were lysed by adding 10 µl of detection mix containing cell lysis buffer, and then allowed for a reaction at room temperature for 90 minutes. The cell lysate obtained by the completion of the reaction was applied to the LANCE cAMP kit (PerkinElmer, USA) to calculate the EC₅₀ value through the accumulated cAMP to be compared with each other.

Relative potencies compared to human GIP in the human GIP receptors are shown in Table 2.

**Table 2**

| SEQ ID NO. | *In vitro* activity of GIP derivative compared to human native GIP in human GIP receptor (%) |
|---|---|
| 1 | 49.3 % |
| 2 | 13.9 % |
| 3 | 15.6 % |
| 4 | 10.5 % |
| 5 | 12.1 % |
| 6 | 17.4 % |
| 7 | 19.6% |
| 8 | 1.9 % |
| 9 | 2.0 % |
| 10 | 13.9 % |
| 11 | 121.9 % |
| 12 | 49.2 % |
| 13 | 31.1 % |
| 14 | 17.4 % |
| 15 | 31.1 % |
| 16 | 11.3 % |
| 17 | 111.4 % |
| 18 | 13.6 % |
| 19 | 66.7 % |
| 20 | 75.3 % |
| 21 | 122.7 % |
| 22 | 71.6 % |
| 23 | 87.6 % |
| 24 | 183.0 % |
| 25 | 78.3 % |
| 26 | 66.7 % |

### Example 3: Preparation of long-acting GIP conjugate

A long-acting conjugate including the GIP derivative prepared in Example 1 was prepared. In detail, the GIP derivatives of SEQ ID NOs: 11, 17, 21 and 24 were each linked to an immunoglobulin Fc region through PEG which is a non-peptide polymer.

A specific manufacturing process of the long-acting conjugate is as follows, and the same process was repeated to prepare GIP derivative conjugates of SEQ ID NOs: 11, 17, 21 and 24. For pegylation at the N-terminus of the immunoglobulin Fc region, the immunoglobulin Fc region and MAL-10K PEG-ALD (10 kDa PEG having a maleimide group and a propionaldehyde group, respectively, at both ends, NOF, Japan) were reacted at a molar ratio of 1:1 to 2 at a total protein concentration in a range of 40 mg/ml to 60 mg/ml at a pH in a range of 6.0 to 6.5 for about 3 hours to about 4 hours at a temperature in a range of 4 °C to 8 °C. Here, the reaction was carried out by adding sodium cyanoborohydride (NaCNBH₃) as a reducing agent, and reaction solution was subjected to the CaptoQ ImpRes (GE Healthcare Life Science, USA) column to purify mono-pegylated immunoglobulin Fc region.

Then, to link the purified mono-pegylated immunoglobulin Fc region to the GIP derivative, the mono-pegylated immunoglobulin Fc region and the GIP derivatives (SEQ ID NOs: 11, 17, 21, and 24) were reacted at a molar ratio of 1:1 to 3,at a total protein concentration in a range of 0.1 mg/ml to 0.5 mg/ml in an isopropanol-containing buffer for about 14 hours to about 18 hours at a temperature in a range of 4 °C to 8 °C. The reaction solution was subjected to the Source 15ISO (GE Healthcare Life Science, USA) column to purify conjugates in which the GIP derivatives (SEQ ID NO: 11, 17, 21, and 24) were each covalently linked to the immunoglobulin Fc region by PEG.

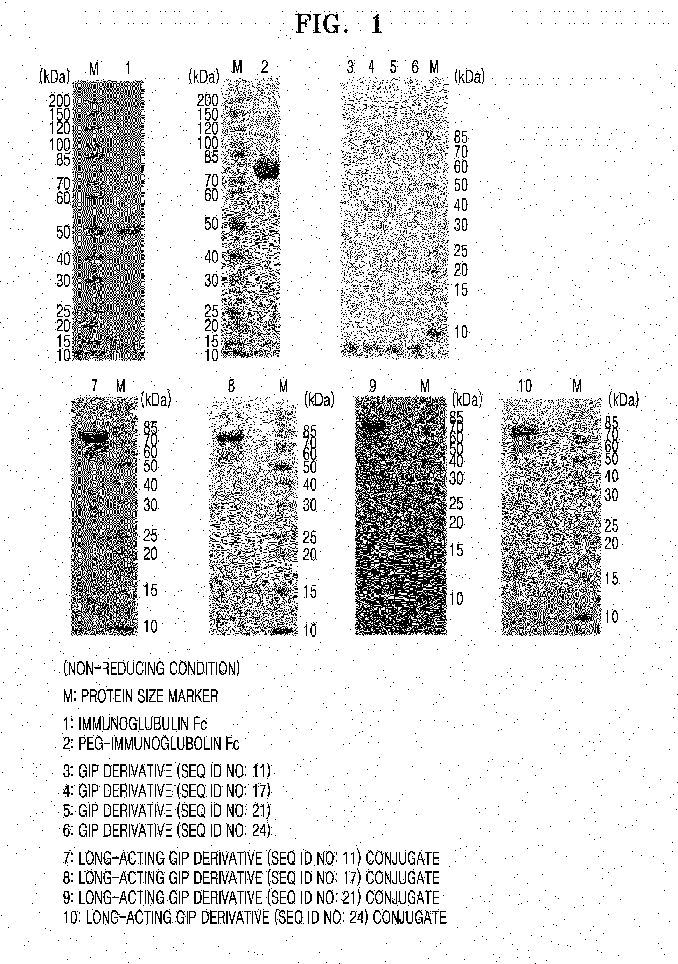

As a result, a conjugate of purified GIP derivative of SEQ ID NO: 11-PEG-immunoglobulin Fc region, a conjugate of purified GIP derivative of SEQ ID NO: 17-PEG-immunoglobulin Fc region, a conjugate of purified GIP derivative of SEQ ID NO: 21-PEG-immunoglobulin Fc region, and a conjugate of purified GIP derivative of SEQ ID NO: 24-PEG-immunoglobulin Fc region were found to be prepared with a high purity of 90 % or more, and results of SDS-PAGE analysis are shown in FIG. 1.

### Example 4: Measurement of in vitro activity of long-acting GIP conjugate

To measure the activity of the long-acting GIP conjugates prepared in Example 3, a method of measuring cell activity *in vitro* using a cell line transformed with the GIP receptor was used in the same manner as in Example 2.

In detail, to measure the activity of the long-acting GIP derivatives in the human GIP receptors, human GIP was serially diluted from 16 nM to 0.000015 nM by 4 folds, and the long-acting GIP derivatives prepared in Example 1 were each serially diluted from 50 nM to 0.000048 nM by 4 folds. The culture medium was removed from the cultured CHO cells where the human GIP receptors were expressed, 5 µl of each of the serially diluted materials was added to the cells, and then, 5 µl of buffer containing cAMP antibody was added thereto, followed by incubation at room temperature for 15 minutes. Next, the cells were lysed by adding 10 µl of detection mix containing cell lysis buffer, and then allowed for a reaction at room temperature for 90 minutes. The cell lysate obtained by the completion of the reaction was applied to the LANCE cAMP kit (PerkinElmer, USA) to calculate the EC₅₀ value through the accumulated cAMP to be compared with each other.

Relative potencies compared to human GIP in the human GIP receptors are shown in Table 3.

**Table 3**

| SEQ ID NO. | *In vitro* activity of long-acting GIP derivative compared to human native GIP in human GIP receptor (%) |
|---|---|
| 11 | 84.8 % |
| 17 | 153.2 % |
| 21 | 148.5 % |
| 24 | 123.3 % |

In this Example, it was confirmed that the GIP derivative of the present disclosure retains the activity of native GIP, and especially when prepared in the form of the long-acting conjugate, not only the activity equivalent to or higher than that of native GIP, but also the increased half-life were exhibited, thereby showing excellent properties of the long-acting GIP derivative as a drug.

### Example 5: Confirmation of efficacy of long-acting GIP conjugate in lupus-associated disease model

To confirm efficacy of the long-acting GIP derivative on lupus in a disease model, MRL/Ipr mice were used.

A normal mouse control group administered with an excipient and a control group of disease model with an excipient were prepared, as well as test groups each administered with the long-acting GIP conjugate in a concentration of 0.12 mg/kg, 1.05 mg/kg, and 3.16 mg/kg. The administration of the excipient and the drugs was performed at 2-day intervals, and the experiment was terminated at the 10th week. For use as the long-acting GIP conjugate, the long-acting GIP conjugate (SEQ ID NO: 17) prepared in Example 3 was used.

The body weight of mice was measured during the administration period, and the degree of skin lesions was measured by observing the muzzle, ears, wing bones, and eyes at intervals of one week from the 4th week and recorded in scores. After the experiment was completed, the spleen was obtained by autopsy, and the weight was measured and observed.

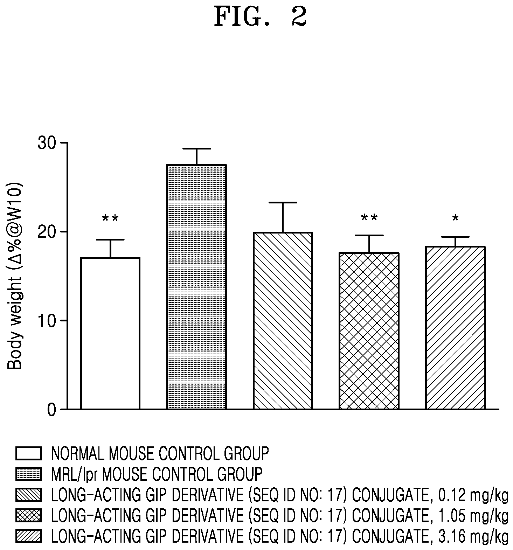

FIG. 2 is a graph showing the weight change rate (%) over 10 weeks in the normal mouse control group, the disease model mouse (MRL/Ipr) control group, and the group administered with a long-acting GIP conjugate.

As shown in FIG. 2, it was confirmed that the disease model mice had increased body weight compared to the normal mice. In the case of lupus patients, body weight is increased due to systemic edema caused by an inflammatory response, and likewise, the disease model also showed the same characteristics. In addition, in the case of the test group administered with the long-acting GIP conjugate, it was confirmed that the body weight of the test group was reduced compared to that of the disease model mouse control group, thereby confirming that edema was ameliorated.

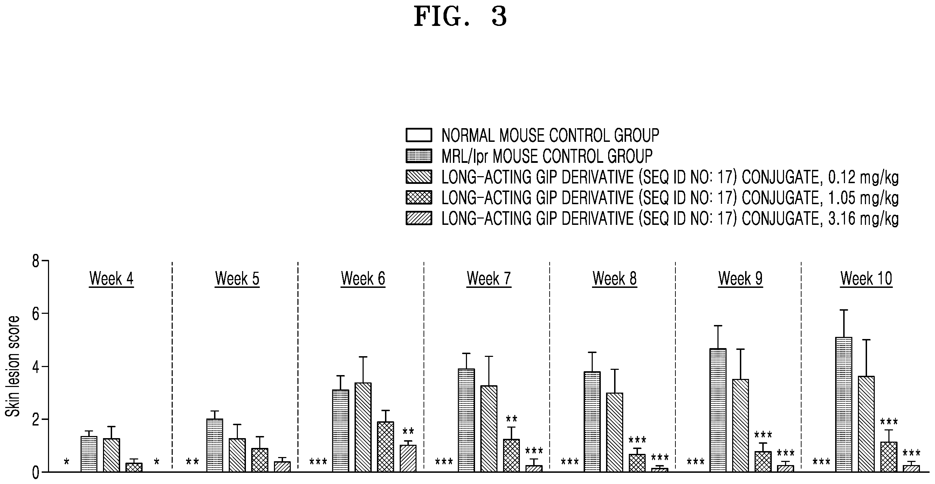

FIG. 3 is a graph showing the skin lesion scores after 10 weeks of administration in the normal mouse control group, the disease model mouse (MRL/Ipr) control group, and the group administered with a long-acting GIP conjugate.

As shown in FIG. 3, it was confirmed that the skin lesions in the disease model mouse control group was gradually worsened over time. However, in the case of the group administered with the long-acting GIP conjugate, the degree of skin lesions was restored close to that of the normal mice.

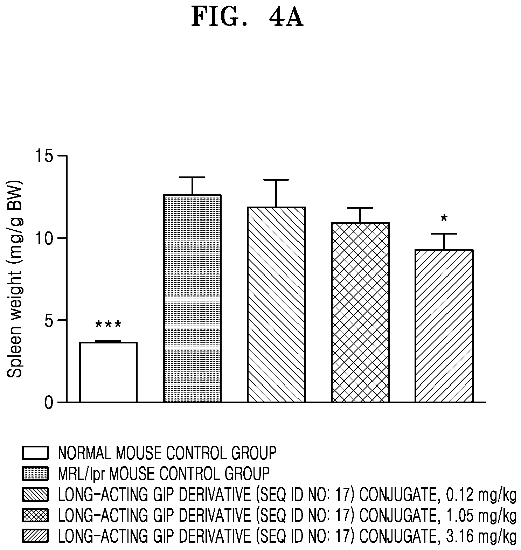

FIG. 4A is a graph showing the spleen weight after 10 weeks of administration in the normal mouse control group, the disease model mouse (MRL/Ipr) control group, and the group administered with a long-acting GIP conjugate.

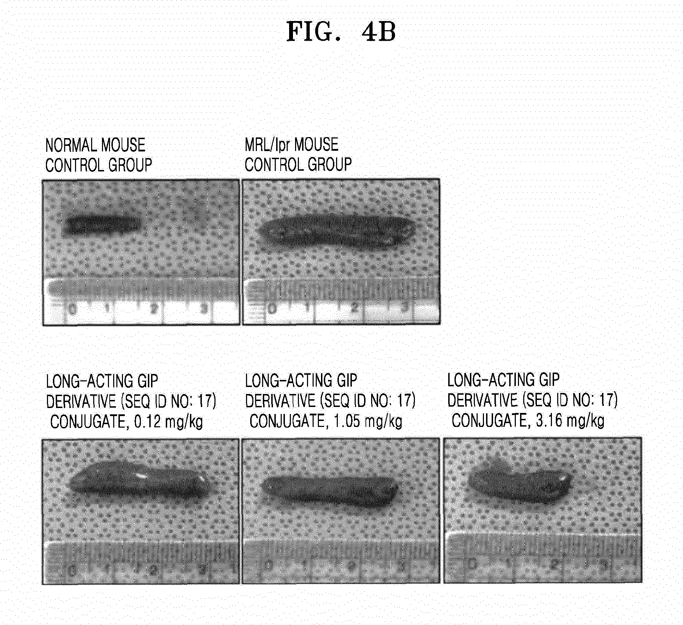

FIG. 4B shows the spleen size after 10 weeks of administration in the normal mouse control group, the disease model mouse (MRL/Ipr) control group, and the group administered with a long-acting GIP conjugate.

As shown in FIGS. 4A and 4B, the disease model mouse control group showed significant splenomegaly due to an inflammatory reaction. Meanwhile, in the case of the group administered with the long-acting GIP conjugate, the weight and size of the spleen decreased compared to the disease model mouse control group.

Accordingly, it was found that the long-acting GIP conjugate has an effect of treating or improving lupus-associated disease.

## Claims

1. A pharmaceutical composition for preventing or treating lupus-associated disease, comprising
a pharmaceutically acceptable excipient and a pharmaceutically effective amount of a peptide comprising the amino acid sequence of any one of SEQ ID NOs: 1 to 26.

2. The pharmaceutical composition of claim 1, wherein the peptide is in the form of a long-acting conjugate, and the long-acting conjugate is represented by Formula 1:
Formula 1 X-L-F
wherein, X is a peptide comprising the amino acid sequence of any one of SEQ ID NOS: 1 to 26,
L is a linker containing an ethylene glycol repeating unit,
F is an immunoglobulin Fc region, and
- represents a covalent bond linkage between X and L and a covalent bond linkage between L and F.

3. The pharmaceutical composition of claim 1 or claim 2, wherein the peptide of which a C-terminus is amidated.

4. The pharmaceutical composition of claim 1, wherein the peptide comprises the amino acid sequence selected from the group consisting of SEQ ID NOs: 11, 17, and 19 to 26.

5. The pharmaceutical composition of claim 4, wherein the peptide comprises the amino acid sequence selected from the group consisting of SEQ ID NOs: 11, 17, 21, and 24.

6. The pharmaceutical composition of claim 2, wherein the ethylene glycol repeating unit included in L has a formula weight in a range of 1 kDa to 100 kDa.

7. The pharmaceutical composition of claim 2, wherein F indicates an IgG Fc region.

8. The pharmaceutical composition of claim 1 or claim 2, wherein the lupus-associated disease is **characterized by** the appearance of symptoms in any one or more organs among the lungs, heart, muscles, and joints.

9. The pharmaceutical composition of claim 1 or claim 2, wherein the lupus-associated disease is systemic lupus erythematosus (SLE), discoid lupus erythematosus (DLE), drug-induced lupus, neonatal lupus, lupus nephritis, butterfly rash, or pernio-like rash.

10. The pharmaceutical composition of claim 1 or claim 2, wherein the pharmaceutical composition reduces skin lesions.
